# EUROPEAN PATENT APPLICATION

(11) **EP 2 112 509 A1**
(43) Date of publication of application: **28.10.2009**
(21) Application number: 08005503.1
(22) Date of filing: 25.03.2008
(51) Int. Cl.: G01N 33/50, C12Q 1/34

(54) **Centrosome-assay**

(71) Applicant: 4SC AG, 82152 Planegg - Martinsried (DE)
(72) Inventor: The designation of the inventor has not yet been filed
(74) Representative: Vossius & Partner

(57) **Abstract**

The present invention provides a centrosome assay for the identification of, preferably, Eg5 inhibitors.

## Description

The present invention pertains to a method for identifying a compound which has an effect on mitosis, to the respective method of screening and the compound obtained thereby, and to identifying a compound as a cellular proliferation protein acting on mitosis.

In particular, the present invention relates to a method of identifying a compound as an Eg5 inhibitor.

### Background

Mitosis is the process by which a cell duplicates the chromosomes in its cell nucleus, in order to generate two, identical, daughter nuclei. It is generally followed immediately by cytokinesis, which divides the nuclei, cytoplasm, organelles and cell membrane into two daughter cells containing roughly equal shares of these cellular components. Mitosis and cytokinesis together define the mitotic (M) phase of the cell cycle, the division of the mother cell into two daughter cells, each with the genetic equivalent of the parent cell.

Mitosis occurs exclusively in eukaryotic cells, but occurs in different ways in different species. For example animals undergo an "open" mitosis, where the nuclear envelope breaks down before the chromosomes separate, while yeast such as Saccharomyces cerevisiae and fungi such as Aspergillus nidulans undergo a "closed" mitosis, where chromosomes divide within an intact cell nucleus. In multicellular organisms, the somatic cells undergo mitosis, while germ cells - cells destined to become sperm in males or ova in females - divide by a related process called meiosis. Prokaryotic cells, which lack a nucleus, divide by a process called binary fission.

The process of mitosis is complex and highly regulated. The sequence of events is divided into phases, corresponding to the completion of one set of activities and the start of the next. These stages are prophase, prometaphase, metaphase, anaphase and telophase. During the process of mitosis the pairs of chromosomes condense and attach to fibers that pull the sister chromatids to opposite sides of the cell. The cell then divides in cytokinesis, to produce two identical daughter cells.

Because cytokinesis usually occurs in conjunction with mitosis, "mitosis" is often used interchangeably with "mitotic phase". However, there are many cells where mitosis and cytokinesis occur separately, forming single cells with multiple nuclei. This occurs most notably among the fungi and slime moulds, but is found in various different groups. Even in animals, cytokinesis and mitosis may occur independently, for instance during certain stages of fruit fly embryonic development. Errors in mitosis can either kill a cell through apoptosis or cause mutations that may lead to cancer.

The primary result of mitosis is the division of the parent cell's genome into two daughter cells. The genome is composed of a number of chromosomes, complexes of tightly-coiled DNA that contain genetic information vital for proper cell function. Because each resultant daughter cell should be genetically identical to the parent cell, the parent cell must make a copy of each chromosome before mitosis. This occurs during S phase, in interphase, the period that precedes the mitotic phase in the cell cycle where preparation for mitosis occurs.

Each new chromosome now contains two identical copies of itself, called sister chromatids, attached together in a specialized region of the chromosome known as the centromere. Each sister chromatid is not considered a chromosome in itself, and a chromosome does not always contain two sister chromatids.

In most eukaryotes, the nuclear envelope that separates the DNA from the cytoplasm disassembles. The chromosomes align themselves in a line spanning the cell. Microtubules, essentially miniature strings, splay out from opposite ends of the cell and shorten, pulling apart the sister chromatids of each chromosome. As a matter of convention, each sister chromatid is now considered a chromosome, so they are renamed to sister chromosomes. As the cell elongates, corresponding sister chromosomes are pulled toward opposite ends. A new nuclear envelope forms around the separated sister chromosomes.

As mitosis completes cytokinesis is well underway. In animal cells, the cell pinches inward where the imaginary line used to be, (the pinching of the cell membrane to form the two daughter cells is called cleavage furrow) separating the two developing nuclei. In plant cells, the daughter cells will construct a new dividing cell wall between each other. Eventually, the mother cell will be split in half, giving rise to two daughter cells, each with an equivalent and complete copy of the original genome.

Prokaryotic cells undergo a process similar to mitosis called binary fission. However, prokaryotes cannot be properly said to undergo mitosis because they lack a nucleus and only have a single chromosome with no centromere.

The importance of mitosis is the maintenance of the chromosomal set; each cell formed receives chromosomes that are alike in composition and equal in number to the chromosomes of the parent cell. Transcription is generally believed to cease during mitosis, but epigenetic mechanisms such as bookmarking function during this stage of the cell cycle to ensure that the "memory" of which genes were active prior to entry into mitosis are transmitted to the daughter cells.

Eg5 (also called kinesin spindle protein Ksp) is a protein responsible for the formation of the mitotic spindle during cell division, with a centrosome at each of the two edges of the spindle.

Kinesins are a class of motor proteins found in eukaryotic cells. Kinesins move along microtubule cables powered by the hydrolysis of ATP (thus kinesins are ATPases). The active movement of kinesins supports several cellular functions including mitosis, meiosis and transport of cargo such as axonal transport.

Members of the kinesin family vary in shape but the typical kinesin is a protein dimer (molecule pair) consisting of two heavy chains and two light chains. The heavy chain comprises a globular head (the motor domain) connected via a short, flexible neck linker to the stalk - a long, central coiled-coil region - that ends in a tail region formed with a light-chain. The stalks intertwine to form the kinesin dimer. Cargo binds to the tail while each head has two separate binding sites: one for the microtubule and the other for ATP. ATP binding and hydrolysis as well as ADP release change the conformation of the microtubule-binding domains and the orientation of the neck with respect to the head: this results in the motion of the kinesin. Several structural elements in the head, including a central beta-sheet domain and the Switch I and II domains, have been implicated as mediating the interactions between the two binding sites and the neck domain. Kinesins are related structurally to G-proteins, which hydrolyze GTP instead of ATP. Several structural elements are shared between the two families, notably the Switch I and Switch II domains.

Motor proteins travel in a specific direction along a microtubule. This is because the microtubule is polar, the heads only bind to the microtubule in one orientation, and ATP hydrolysis drives the molecule in one direction.

Most kinesins walk towards the positive end of a microtubule which, in most cells, entails transporting cargo from the centre of the cell towards the periphery. This form of transport is known as anterograde transport.

Some kinesins, like EG5 and a different type of motor protein known as dyneins, move towards the minus end of the microtubule. Thus they transport cargo from the periphery of the cell towards the centre. This is known as retrograde transport. However, dyneins are the much faster and more ubiquitous of the microtubule-binding retrograde transporters.

In recent years, it has been found that microtubule-based molecular motors (including a number of kinesins) have a role in mitosis (cell division). The mechanism by which the cytoskeleton of the daughter cell separates from that of the mother cell was unclear. It seems that motors organize the two separate microtubule asters into a metastable structure independent of any external positional cues. This self-organization is in turn dependent on the directionality of the motors as well as their processivity ("ability to walk"). Thus motors are necessary for the formation of the mitotic spindle assemblies that perform chromosome separation.

Inhibition of Eg5 leads to so-called "mono-asters", the spindle materials directed towards the outside starting from the centrosome in the center in a ball-like manner and the centrosomes showing a more diffuse shape than in undisturbed cell division.

In view of the above it would be desirable to have an assay which would be capable of identifying a compound which has an effect on mitosis, either as a possible mitosis effector or inhibitor or as a new target. In particular it would be desirable to have an assay which could identify a compound which is antimitotic.

The above-mentioned objects have been solved according to the present centrosome assay for identification of compounds which have an effect on mitosis.

According to one embodiment a method for identifying a compound which has an effect on mitosis is provided, by contacting cells with the candidate compound, comprising the further steps:
(1) detection of mitotic cells
(2) detection of cells with an altered centrosome number and/or morphology, by staining of centrosomes.

In particular the present inventors developed in a preferred embodiment an assay wherein cancer cells, that are grown in 96 well-plates and that have been treated with the prospective Eg5 inhibitor compounds, are fixed and submitted to a sequence of staining: (1) DNA staining (detection of space where a cell is), (2) Phospho Histone H3 staining (detection of mitotic cells among the cells) and (3) staining of pericentrine (detection of chromosomes). The result is read out by a commercially available machine that takes fluorescence pictures and interprets the picture, depending on the settings the user defines. The machine is able to analyze automatically the intensity of fluorescence as well as the pattern of the intensity of fluorescence (by definition of area). The software is included in the machine. The settings can be adapted by the user to meet the requirements of the parameters to be measured; these adaptations are within the reach of an ordinary person skilled in the art.

The sequence of staining leads to a selection of subpopulations of cells: first, cells versus non-cell space is selected. Secondly, mitotic cells are selected among the cells, using their characteristic pattern. At this stage, the mitotic index can optionally be determined.

In the third step, it is distinguished between cells that show two distinct centrosomes within a cell (mitotic, but "un-inhibited" cells, e.g. untreated control cells) and the "cloudy"-looking Eg5-inhibited cells that are recognized by their pattern defined by the parameters (a) number of centrosomes: only 1, and (b) size and intensity of the fluorescent area. The pattern of the cell morphology therefore is used to screen for the effect of compounds, which can be done in an automated manner after initial optimization of settings. The possibility of automatization is an important aspect in this invention.

Performing the assay at different concentrations, a dose-effect relationship curve can be obtained, and an EC₅₀ value can be determined.

In a further embodiment, the invention pertains to identifying a compound as a cellular protein acting on mitosis, preferably a cellular proliferation protein, by contacting cells expressing said protein with the candidate compound, and carrying out the staining steps as described above.

Preferably, the cellular protein is a cellular proliferation protein which can be an antimitotic agent. The antimitotic agent according to a preferred embodiment is a mitotic kinesin or mitotic kinase.

In particularly preferred embodiments, the cellular proliferation protein is Eg5, Plk1 or HDAC. In the most preferred embodiment the centrosome assay according to the present invention can be used for the identification of Eg5 inhibitors.

Step (1) as mentioned above, which is the detection of mitotic cells, can be divided into two sub-steps, 1(a) and 1(b). In a first sub-step 1(a) a space is detected where the cells are located, while in a second sub-step 1(b), a sub-group of mitotic cells among the cells detected in substep 1(a) are detected.

The detection according to sub-step 1(a), i.e. the detection of the space where cells are actually located can be carried out preferably by a DNA staining. Any DNA staining would be possible; preferably, it can be carried out by the so-called Hoechst stain.

The Hoechst stains are part of a family of fluorescent stains for labelling DNA in fluorescence microscopy. Because these fluorescent stains label DNA, they are also commonly used to visualize nuclei and mitochondria. Two of these closely related bis-benzimides are commonly used: Hoechst 33258 and Hoechst 33342.

Both dyes are excited by ultraviolet light at around 350 nm, and both emit blue/cyan fluorescence light around an emission maximum at 461 nm. The Hoechst stains may be used on live or fixed cells, and are often used as a substitute for another nucleic acid stain, DAPI. The key difference between them is that the additional ethyl group of Hoechst 33342 renders it more lipophilic, and thus more able to cross intact cell membranes. In some applications, Hoechst 33258 is significantly less permeant. However, the DAPI stain is suitable in the present context as well.

These dyes can also be used to detect the contents of a sample DNA by plotting a standard emission-to-content curve.

As mentioned above, the principle of the Hoechst stain is based on fluorescence.

Fluorescence is a luminescence that is mostly found as an optical phenomenon in cold bodies, in which the molecular absorption of a photon triggers the emission of another photon with a longer wavelength. The energy difference between the absorbed and emitted photons ends up as molecular vibrations or heat. Usually the absorbed photon is in the ultraviolet range, and the emitted light is in the visible range, but this depends on the absorbance curve and Stokes shift of the particular fluorophore. Fluorescence is named after the mineral fluorite, composed of calcium fluoride, which often exhibits this phenomenon.

Fluorescence in several wavelengths can be detected by an array detector, to detect compounds from e.g. HPLC flow. Also, TLC plates can be visualized if the compounds or a colouring reagent is fluorescent. Fluorescence is most effective when there is a larger ratio of atoms at lower levels in a Boltzman distribution because then there is more of a chance those atoms will be excited then release a photon and can be analyzed.

The detection of the mitotic cells among the cells which have been detected as in sub-step 1(a) can be carried out by any suitable staining, preferably a phospho histone H3 staining in sub-step 1(b).

Histone H3 as a mitosis marker is specific for phosphorylated histone H3, Mr 17 kDa, and has been reported to detect mitotic cells for example in C. elegans and Drosophila embryos by immunohistochemistry.

A phospho histone H3 antibody can be used, which is commercially available.

In a preferred embodiment, the detection according to step (2), i.e. the detection of cells with an altered centrosome number and/or morphology, by staining of centrosomes, can be carried out by pericentrin staining.

Pericentrin (kendrin), also known as PCNT, is a human gene.

The protein encoded by this gene binds to calmodulin and is expressed in the centrosome. It is an integral component of the pericentriolar material (PCM). The protein contains a series of coiled-coil domains and a highly conserved PCM targeting motif called the PACT domain near its C-terminus. The protein interacts with the microtubule nucleation component gamma-tubulin and is important to normal functioning of the centrosomes, cytoskeleton, and cell-cycle progression.

Pericentrin stainings are again commercially available and known to a person skilled in the art.

Very preferably, the final result is read out by a device taking fluorescent pictures. This device is again preferably an automatic analyser of the intensity of fluorescence as well as pattern of intensity of fluorescence.

The preferred cells to be used according to the method as described above are H460 cells.

H460 cells are a human lung carcinoma cell line. This cell line e.g. is available from ATCC.

In a preferred embodiment, the method can be sequentially repeated. Even more preferably, the analysis and selection are automated; here, the specific concentrations of a candidate compound are used and their effect is determined. According to a specifically preferred embodiment, the method is sequentially repeated at at least two different, preferably at least three different, concentrations of the candidate compound so as to determine a dose activity relationship or inhibitory concentration.

In particular, the method can be a method for determination of the binding of a candidate compound to a cellular protein which has an effect on mitosis, wherein the method is characterised by the features as defined above.

Further, the method can be a method of screening for a cellular protein which has an effect on mitosis, wherein the method is defined according to the features above.

An especially preferred embodiment is a method for identifying a compound as an Eg5 inhibitor, by contacting H460 cells with a candidate compound, and comprising the steps of:
(1a) detection of cells by staining of DNA,
(1b) detection of mitotic cells by phospho-Histon-H3 staining with Anti-Phospho-Histon-H3 antibody, optionally followed by determination of the mitotic index,
(2) detection of inhibited cells by staining of the centrosomes with anti-pericentrine and detecting the number and morphology of the centrosome or centrosomes, and
(3) quantification of number and morphology of the centrosome or centrosomes, optionally automated by a computer-assisted algorithm.

This centrosome assay for the identification of Eg5 inhibitors is a cellular Eg5 inhibitor specific assay, where the centrosome number and morphology in mitotic cells is detected quantitatively.

In a preferred embodiment, H460 cells are seeded onto 96 well plates. After approximately 24 hours the cells are treated in a CO₂ incubator at 37° Celsius with different concentrations of the substance to be tested. Thereafter, the cells are fixed and stained as follows:
DNA stain according to Hoechst for the detection of cells/chromosomes.
Antipericentrin antibody for the staining of centrosomes and Anti-phospho Histon H3 antibody for the identification of mitotic cells.
Anti-pericentrin antibody and anti-phospho histon H3 antibody are detected with a secondary antibody which are labelled with a fluorescent dye.
Using a High Content screening device, known to a person skilled in the art, automatic fluorescence images are taken from the stained cells for each of the used dyes. The evaluation of the images is carried out for example with a compartmental analysis algorithm (available from Cellomics, "Array Scan"). A subpopulation analysis of the phospho-histon H3 positive (mitotic cells) is carried out.

The following parameters are determined:
- rate of mitotic cells from the whole cell count
- percentage of mitotic cells with one centrosome (number of centrosomes)
- size of centrosomes.

As criteria for the identification of Eg5 inhibitors, the following are selected:

The centrosome size should be bigger than that of a negative control.

A lower percentage limit of mitotic cells with only one centrosome is determined.

The EC₅₀ value for the compounds to be tested are determined for example with the programme "Graph Pad Prism" (available from Graph Pad Software).

The present invention also pertains to a compound or cellular protein which has an effect on mitosis obtained by the method described above and the use of the compound or cellular protein as an antimitotic agent or as a target.

One particular Eg5 inhibitor is monastrol, a cell-permeable inhibitor of the kinesin Eg5, which has been used to probe the dynamic organization of the mitotic spindle. It has been found that monastrol inhibits both the basal and the microtubule-stimulated ATPase activity of the Eg5 motor domain. Unlike many ATPase inhibitors, monastrol does not compete with ATP binding to Eg5. Monastrol appears to inhibit microtubule-stimulated ADP release from Eg5 but does not compete with microtubule binding, suggesting that monastrol binds a novel allosteric site in the motor domain. It was established that (S)-monastrol, as compared to the (R)-enantiomer, is a more potent inhibitor of Eg5 activity in vitro and in vivo.

### Description of Figures

The following figures are enclosed for further reference:

### Figure 1

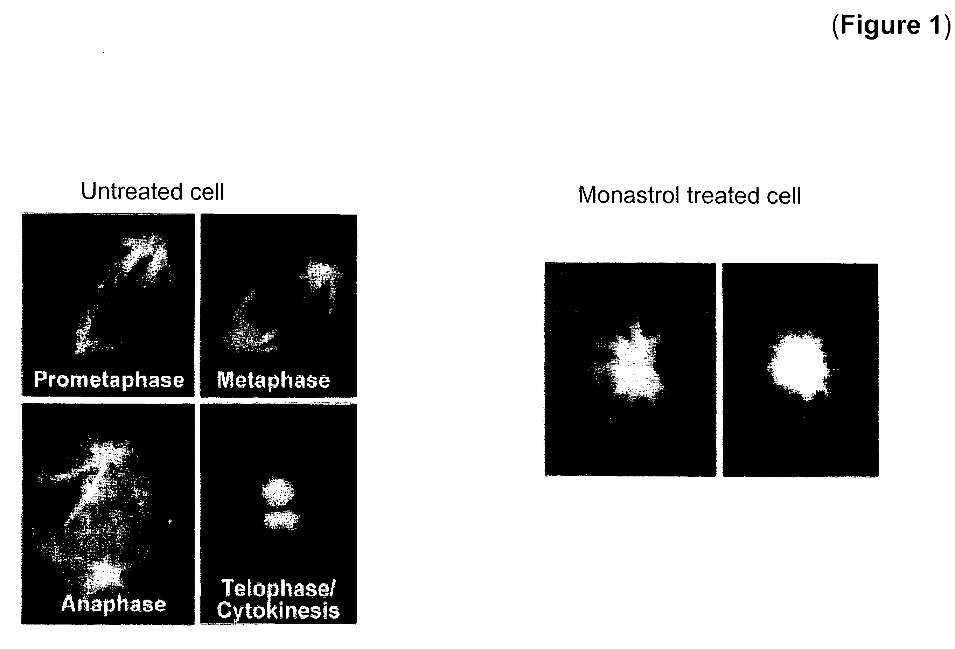

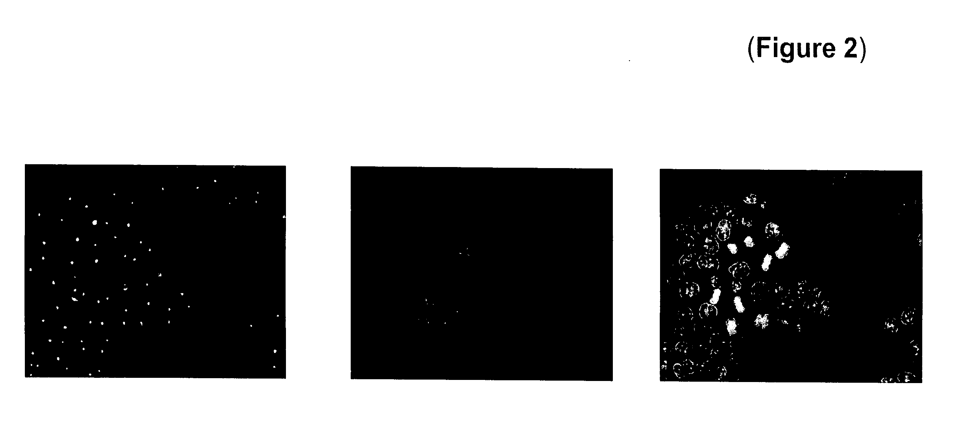

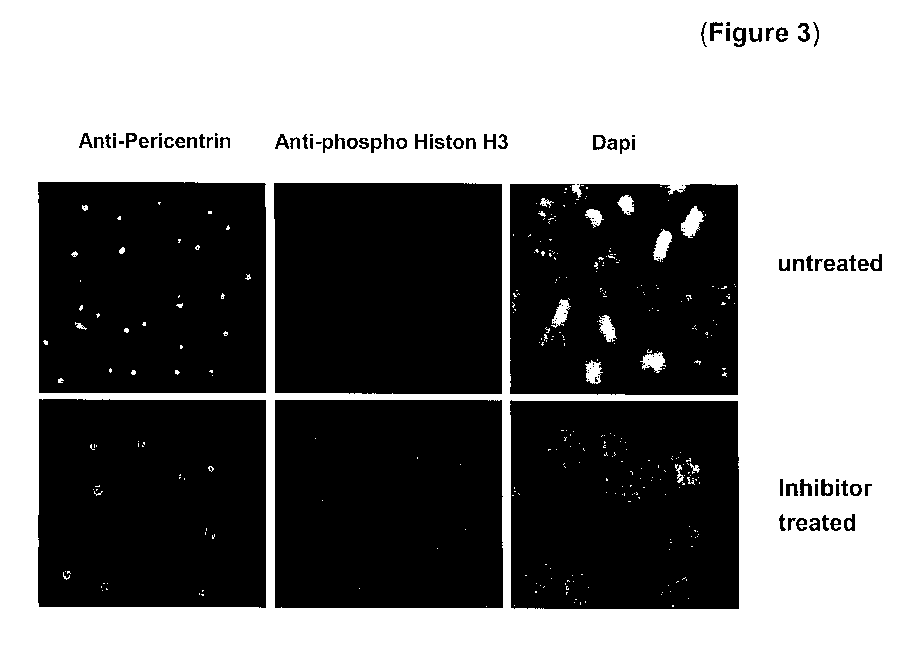

In figure 1, the several mitotic phases, i.e. prometaphase, metaphase, anaphase and telophase/cytokinesis of an untreated H460 cell are shown. Further, these are compared with a cell which has been treated with monastrol. These cells show the typical monoaster phenotype. The DNA staining is shown in dark grey and the microtubule staining is in light grey.

### Figure 2

The staining of relevant cell structures and markers is shown.

On the lefthand side, staining with pericentrin is shown.
The pericentrin is located in/around centrosomes. It enables detection of centrosome number, localization and morphology. In the middle, phospho Histon H3 staining is shown. Phospho Histon H3 is associated with DNA. Positive cells are mitotic.

Finally, on the right side, a DNA stain is given which shows the morphology of nucleus and localization of chromosomes.

### Figure 3

The detection of an Eg5 inhibitor specific phonotype is shown. On the left, again the anti-pericentrin staining is shown, in the middle the anti-phospho Histon H3 stain and on the right, the Dapi staining. The upper line shows untreated cells, while the lower one shows Eg5 inhibitor treated cells.

The pericentrin antibody stains the centrosome bright and the signal to noise ratio is suitable for analysis.

### Figure 4

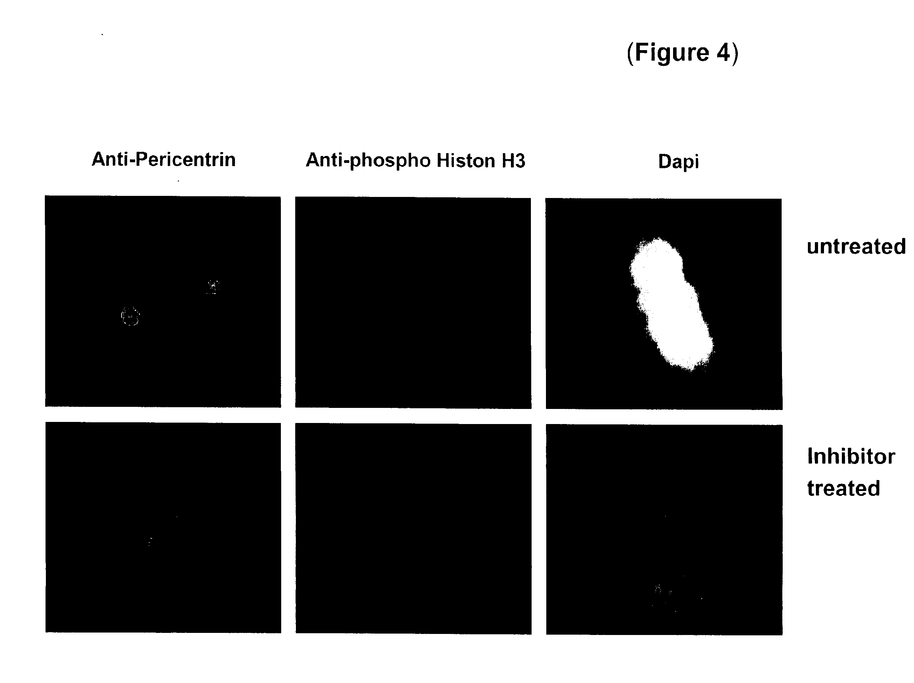

Figure 4 compares the centrosome morphology of untreated versus inhibitor treated cells.

Again, on the lefthand side, anti-pericentrin staining is shown, in the middle anti-phospho Histon H3 staining and on the right Dapi staining. The specific centrosome morphology after treatment with an Eg5 inhibitor, becomes absolutely clear.

The specific morphological characteristics of Eg5 inhibitor-treated cells are that the mitotic cells preferably show one detectable centrosome, and the centrosome in these cells are bigger than in the untreated controls.

### Figure 5

Here the validation of the present cellular Eg5 inhibitor assay is shown.

Several compounds have been tested with regard to maximal percent mitotic cells with one centrosome.

### Figure 6

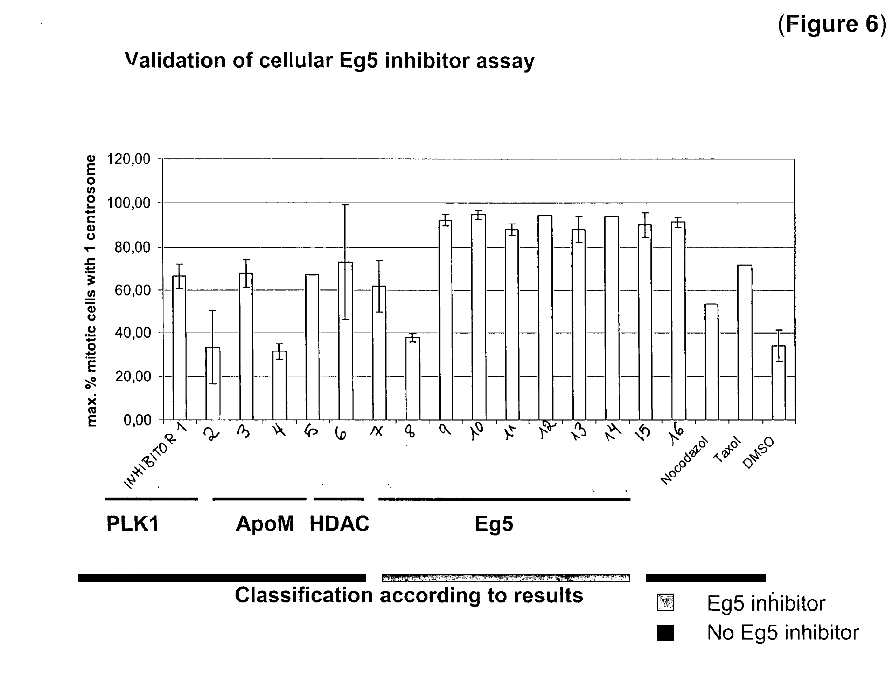

Again, figure 6 pertains to a validation of a cellular Eg5 inhibitor assay, according to the present invention.

Here, a classification according to the results has been carried out so as to determine whether or not a given compound is an Eg5 inhibitor.

### Best embodiment

In the following, the preferred assay procedure is described.

In a first step H460 cells are seeded on collagen-coated plates with 96 wells.

The cells are treated with two or more different compound concentrations of a compound to be tested.

Thereafter, the cells are fixed and stained in subsequent staining steps.

As pointed out above, in a first step a DNA staining is carried out, followed by a phospho Histon H3 staining, which detects mitotic cells and finally a pericentrin staining, which detects centrosomes.

After staining, a specific algorithm like the algorithm "compartmental analysis" is used to detect the structures and to analyse the subpopulation of mitotic cells. In particular it is analysed how many mitotic cells have no, one or two or more centrosomes, and how big the centrosomes are.

Thus, a preferred automated quantification of an Eg5 inhibitor-specific cell morphology is comprised of a determination of centrosome number in mitotic cells, a determination of centrosome size in mitotic cells, the determination of EC₅₀ values reported for Eg5 inhibitors and the determination of the mitotic index, plus, the compartmental analysis algorithm.

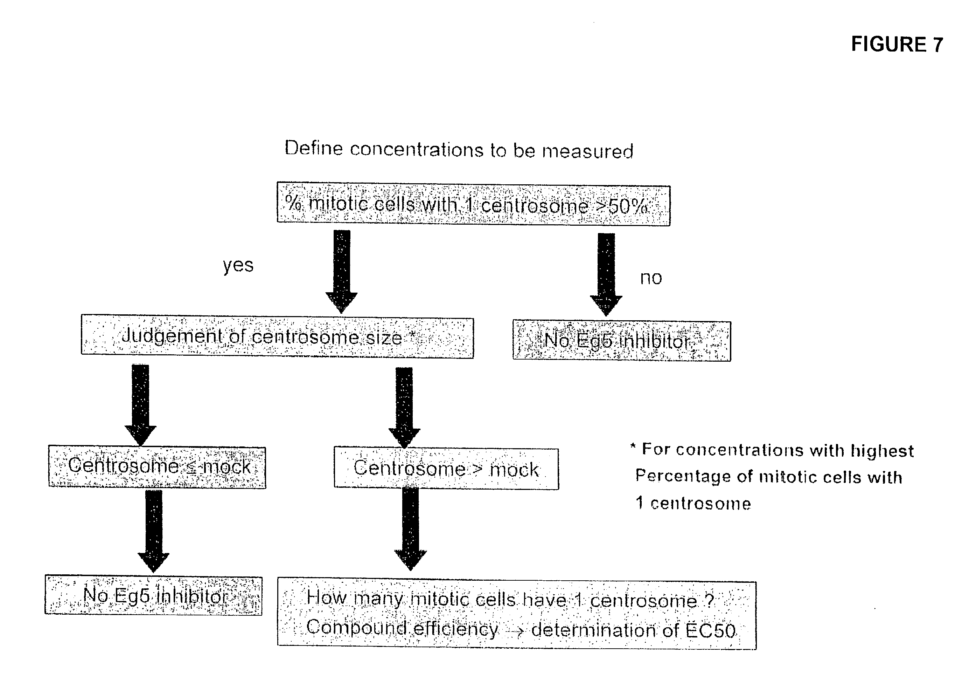

The results can then be classified as follows from appended Figure 7.

### Examples

The present method was carried out by submitting presumed antimitotic compounds to the assay as designed by the present inventors.

To this avail, sixteen presumed antimitotic compounds as well as Nocodazol, Taxol and DMSO were selected; DNA staining was carried out with the above Hoechst stain; thereafter, a phospho histon H3 staining was carried out to detect the mitotic cells among the cells as defined according to the DNA staining step and thereafter the altered centrosome number and/or morphology was detected by pericentrin staining. The cells used were H460 cells (ATCC).

The results were read out by a device taking a fluorescent picture, in this case with the help of the "Array scan" with compartmental analysis algorithm. The EC₅₀ value for the compounds to be tested were determined with the Graph Pad Prism Program.

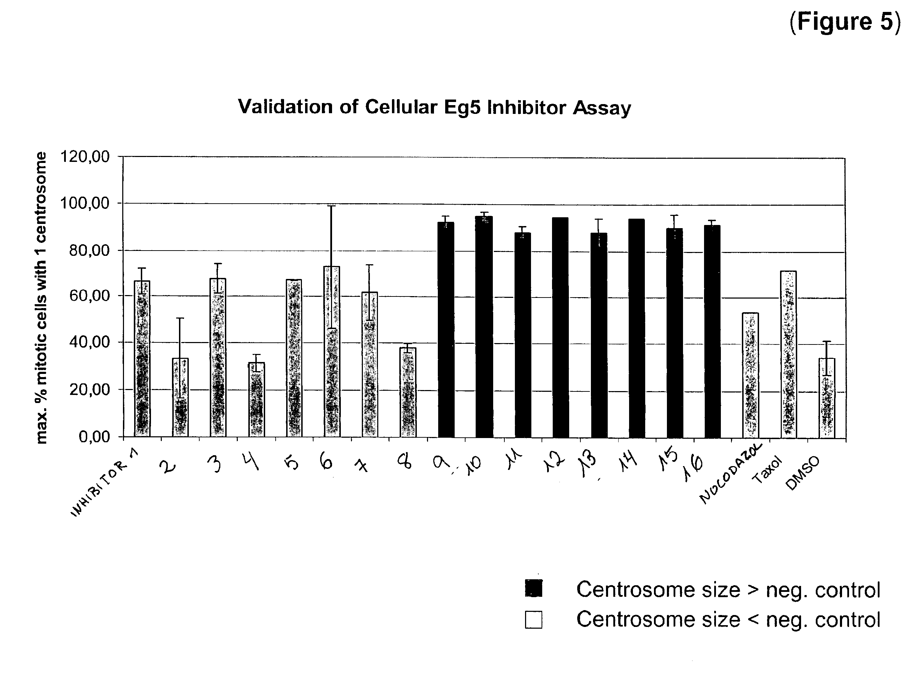

The maximal percentage of mitotic cells with one centrosome were determined and are derivable from Figures 5 and 6 as enclosed herewith.

As can be taken from these results, all presumed Eg5 inhibitors had very high maximal percentages of mitotic cells with one centrosome and could be identified as Eg5 inhibitors.

## Claims

1. A method for identifying a compound which has an effect on mitosis, by contacting cells with a candidate compound, comprising the further steps, - in any sequence -
(1) detection of mitotic cells
(2) detection of cells with an altered centrosome number and/or morphology, by staining of centrosomes.

2. The method of claim 1, wherein the steps are carried out in the sequence as shown in claim 1.

3. The method according to any of claims 1 or 2, wherein the compound is an anti-mitotic agent.

4. The method according to claim 3, wherein the antimitotic agent is a mitotic kinesine or a mitotic kinase.

5. The method according to any of claims 1 to 4, wherein the compound has an effect on a cellular proliferation protein, wherein the cellular proliferation protein is Eg5, Plk1 or HDAC.

6. The method according to any of claims 1 to 5, wherein in step (1) in a first sub-step (1a), a detection of the space where the cells are located, and in a second sub-step (1b), a detection of a sub-group of mitotic cells among the cells is carried out.

7. The method according to any of claims 1 to 6, wherein in step (2) above, inhibited cells are detected, selected from the mitotic cells, by the staining of the centrosome(s) and by detection of number and morphology of the centrosome or centrosomes.

8. The method according to any of claims 1 to 7 which is followed by quantification of the number and morphology of the centrosome or centrosomes (step (3))

9. The method according to any of claims 1 to 8, wherein sub-step (1a) of step (1) is carried out by a DNA staining.

10. The method according to any of claims 1 to 9, wherein the detection of mitotic cells among the cells according to sub-step (1b) is carried out by phospho-Histon-H3-staining.

11. The method according to any of claims 1 to 10, wherein step (2) is carried out by a pericentrin staining.

12. The method according to any of claims 1 to 11, wherein the final result is read out by a device taking fluorescence pictures.

13. The method according to claim 12, wherein the device is automatically analysing the intensity of fluorescence as well as the pattern of the intensity of fluorescence.

14. The method according to any of claims 1 to 13 above, wherein the cells expressing the protein are H460 cells.

15. The method according to any of claims 1 to 14, wherein the method is sequentially repeated.

16. The method according to claim 15, wherein the analysis and selection is automated.

17. The method according to any of claims 1 to 15, wherein the inhibitory concentration of a candidate compound is determined.

18. The method according to claim 15, 16 or 17, wherein the method is sequentially repeated at at least two different concentrations of the candidate compound for determination of the dose activity relationship and/or inhibitory concentration.

19. A method for determination of the binding of a candidate compound to a cellular protein which has an effect on mitosis, wherein the method is **characterised by** the features as defined in any of claims 1 to 18 above.

20. A method of screening for a cellular protein which has an effect on mitosis, wherein the method is defined according to the features as defined in any of claims 1 to 19 above.

21. A method for identifying a compound as an Eg5 inhibitor, by contacting H460 cells with a candidate compound, and comprising the steps of:
(1a) detection of cells by staining of DNA,
(1b) detection of mitotic cells by staining of phospho-Histon-H3 with Anti-Phospho-Histon-H3 antibody, optionally followed by determination of the mitotic index,
(2) detection of inhibited cells by staining of the centrosomes with anti-pericentrine and detecting the number and morphology of the centrosome or centrosomes, and
(3) quantification of number and morphology of the centrosome or centrosomes, optionally automated by a computer-assisted algorithm.

22. A compound which has an effect on mitosis obtained by the method according to any one of claims 1 to 21.

23. The compound according to claim 22 for use as an antimitotic agent.

24. A method of identifying a compound as a cellular proliferation protein acting on mitosis, by contacting cells expressing said protein with the candidate compound, comprising the detection steps as described in any one of the claims above.

25. The method according to claim 1, wherein the cellular protein is a cellular proliferation protein.
